Europäisches Patentamt

European Patent Office

Office européen des brevets

(11) Publication number: **0 071 485**
**B1**

# EUROPEAN PATENT SPECIFICATION

(45) Date of publication of patent specification: **20.09.89**

(21) Application number: **82304039.9**

(22) Date of filing: **30.07.82**

(51) Int. Cl.⁴: **C 12 P 21/02,** C 12 N 1/20 //
C12R1/19, C12N15/00

(54) **Novel microorganisms derived from microorganisms of the genus Escherichia by mutation and their use in the preparation of glutathione.**

(30) Priority: **30.07.81 JP 120544/81**
**30.07.81 JP 120545/81**

(43) Date of publication of application:
**09.02.83 Bulletin 83/06**

(45) Publication of the grant of the patent:
**20.09.89 Bulletin 89/38**

(84) Designated Contracting States:
**DE FR GB IT**

(56) References cited:

PATENTS ABSTRACTS OF JAPAN, vol. 5, no.
152 (C-73)824r, 25th September 1981 & JA - A -
5682099

CHEMICAL ABSTRACTS, vol. 94, no. 13, 30th
March 1981, page 405, no. 99451y, Columbus,
Ohio, US. K. MURATA et al.: "Excretion of
glutathione by methylglyoxal-resistant
Escherichia coli"

(73) Proprietor: **Kimura, Akira**
**80-2, Wakamiya-dori 6-jo-agaru Kami-**
**wakamiya-cho**
**Shimogyo-ku Kyoto-shi Kyoto-fu (JP)**
(73) Proprietor: **Murata, Kousaku**
**3-35, Nishino Hitsugawa-cho Yamashina-ku**
**Kyoto-shi Kyoto-fu (JP)**

(72) Inventor: **Kimura, Akira**
**80-2, Wakamiya-dori, 6-jo-agaru Kami-**
**wakamiya-cho**
**Shimogyo-ku, Kyoto-shi, Kyoto-fu (JP)**
Inventor: **Murata, Kousaku**
**3-35, Nishino, Hitsugawa-cho**
**Yamashina-ku, Kyoto-shi, Kyoto-fu (JP)**
Inventor: **Kato, Jyoji**
**25-16, Otokoyama-yoshii, Yowata-shi**
**Kyoto-fu (JP)**

(74) Representative: **Watkins, Arnold Jack et al**
**European Patent Attorney Frank B. Dehn & Co.**
**Imperial House 15-19 Kingsway**
**London WC2B 6UZ (GB)**

Note: Within nine months from the publication of the mention of the grant of the European patent, any person may
give notice to the European Patent Office of opposition to the European patent granted. Notice of opposition shall
be filed in a written reasoned statement. It shall not be deemed to have been filed until the opposition fee has been
paid. (Art. 99(1) European patent convention).

Courier Press, Leamington Spa, England.

**EP 0 071 485 B1**

(58) References cited:
**CHEMICAL ABSTRACTS, vol. 83, no. 11, 15th September 1975, page 231, no. 92926w, Columbus, Ohio, US. P. APONTOWEIL et al.: "Glutathione biosynthesis in Escherichia coli K 12. Properties of the enzymes and regulation" Applied Environmental Microbiology, vol. 44, pp. 1444-1448 (1982)**

**Description**

The present invention relates to novel microorganisms derived from microorganisms of the genus *Escherichia*, especially *Escherichia coli*, by mutation and their use in the preparation of glutathione.

Glutathione is a tripeptide consisting of the amino acids L-glutamic acid, L-cysteine and glycine and otherwise known as γ - L - glutamyl - L - cysteinylglycine. Glutathione is used *inter alia* as a medicament in the treatment of liver disease, as an antidote and as a biochemical reagent. Conventionally, glutathione is prepared, for example, by extraction from the microbial cells of yeast, by contacting a dried yeast having a high membrane permeability with a substrate solution containing L-glutamic acid, L-cysteine and glycine, or by contacting the microbial cells of yeast or coliform bacilli with the above-mentioned substrate solution. The preparation of glutathione, on an industrial scale, by such known processes however commonly results in a poor yield.

The present invention is based on the discovery that glutathione may be obtained in large amount by the culturing of a microorganism derived by mutation of a bacterium of the genus *Escherichia*, especially *Escherichia coli* and/or by use of an enzyme system obtainable by culturing of said microorganism. The production of large amounts of glutathione by a mutant strain, derived from a wild-type strain of *E. coli*, growing on a medium containing methylglyoxal (MG) has been previously demonstrated (J.G.M. *120*, 545—547, 1980 and Patent Abstracts of Japan, *5* (152), C-73 (824), 25th Sept. 1981). The invention is also based on the microorganisms derivable by mutation which are novel *per se*, and unlike the mutant disclosed in the prior, are cysteine-requiring *and* resistant to MG and 8-hydroxyquinoline.

According to one feature of the present invention there is provided a new mutant strain of *Escherichia coli*, which synthesises a glutathione synthesizing enzyme system comprising γ - glutamyl - L - cysteine synthetase and glutathione synthetase, the said γ - glutamyl - L - cysteine synthetase having a reduced or absent sensitivity to inhibition by glutathione.

According to a further feature of the present invention we provide a process for the preparation of glutathione which comprises culturing a strain as hereinbefore described, said culturing medium effected to accumulate glutathione in the culture medium and recovering the accumulated glutathione therefrom.

The culturing is preferably effected at a pH from 7.0 to 8.5 and at a temperature of from 25 to 37°C for 16—40 hours under aerobic conditions. The glutathione is advantageously recovered by extraction with water from the microbial cells.

Any and all mutant strains induced artificially or naturally from a bacterium of the genus *Escherichia* especially from a wild strain of the genus, for example from a wild strain of *Escherichia coli* may be used for the purpose of the present invention, provided that it is capable of synthesising a glutathione synthesizing enzyme system preferably comprising γ - glutamyl - L - cysteine synthetase (E.C. 6.3.2.2., hereinafter referred to as GSH-I and glutathione synthetase (E.C. 6.3.2.3. hereinafter referred to as GSH-II) e.g. to produce γ - glutamyl - L - cysteine and/or glutathione and provided the GSH-I has a reduced or absent sensitivity to inhibition by glutathione. However, it is preferred to use *Escherichia coli* RC912 (FERM-BP No. 47) deposited at the Fermentation Research Institute. *E. coli* RC912 (FERM-BP No. 47) may be obtained from the Fermentation Research Institute, or this or related strains may for example be produced by subjecting a strain of *E. coli* deficient in the production of GSH-I to mutagenesis and isolating the desired strain by selection in the presence of 8-hydroxyquinoline. Preferably one proceeds in the following manner:—

the induction of a cysteine-requiring strain and a methylglyoxal-resistant strain in a bacterium of the genus *Escherichia* e.g. a wild strain of *E. coli* such as *E. coli* B355[ATCC 23226] by mutation techniques known *per se*, the cysteine requiring strain being obtained by culturing the mutant strains in the presence of L-cysteine followed by isolation of the desired strain, the methylglyoxal-resistant strain being obtained by culturing the mutant strains in the presence of methylglyoxal and isolating the desired strain. The cysteine requiring strain and the methylglyoxal-resistant strain are then mixed and cultured and a mutant strain may be obtained from the culture medium the strain being deficient in the production of GSH-I. The strain is then subjected to mutation followed by culturing in the presence of 8-hydroxy quinoline to obtain the desired mutant strain for use in the present invention.

In more detail the process may be described as follows:—

A wild strain of *Escherichia coli* such as *E. coli* B 355 (ATCC 23226, J. Appl. Biochem. 1, 283, (1979)) comprising the enzyme system GSH-I and GSH-II as hereinbefore defined is treated to induce a cystein-requiring strain and a methyl-glyoxal-resistant strain. The induction of mutant strains may be effected in a conventional manner, for example, by using N-methyl-N'-nitro-N-nitrosoguanidine (NTG), although it is possible to use other mutagens and/or irradiation e.g. by ultraviolet light if desired. After this, a portion of the treated strain is cultured using a minimum medium containing L-cysteine ($2 \times 10^{-5}$ M) and having a composition of $K_2HPO_4$ (0.7%), $KH_2PO_4$ (0.3%), $(NH_4)_2SO_4$ (0.1%), $MgSO_4 \cdot 7H_2O$ (0.01%) and glucose (0.5%) [pH about 7.0; hereinafter referred to as DM medium] at a temperature of 30—37°C for 16—40 hours with shaking to obtain smaller colonies, from which a cysteine-requiring strain may be obtained and cultured pure under the same conditions. Separately, the remaining portion of the strain after induction treatment is cultured under similar conditions using a DM medium containing methyl-glyoxal ($2 \times 10^{-3}$M) to obtain larger colonies, from which a methylglyoxal-resistant strain may be obtained.

The methylglyoxal-resistant strain thus obtained is then transferred to the above-mentioned DM medium containing the cysteine-requiring strain for culturing under the same conditions. In contrast to the majority of MG-resistant cells, some colonies do not produce or excrete glutathione and thus a mutant strain which is GHS-I deficient such as *E. coli* C912 may be obtained from colonies without a halo of cysteine-requiring cells. This strain is then treated with NTG in a similar manner to that described above to induce mutation, followed by culturing under the same conditions as described above by using a DM medium containing 8-hydroxyquinoline ($2 \times 10^{-4}$M) to obtain colonies, from which a mutant strain may be obtained capable of synthesising GSH-I and GSH-II to produce glutathione, wherein the GHS-I has a reduced or absent sensitivity to inhibition by glutathione. The strain thus obtained has been cultured over an extended period of time to confirm that the desired characteristics are stable so that the strain may be used for the purpose of the present invention.

The mutant strains thus obtained may be cultured in conventional manner using an organic medium or synthetic medium containing appropriate amounts of carbon sources, nitrogen sources, inorganic substances and various other substances which may promote the growth of the used strain and enhance the accumulation of glutathione. Examples of carbon sources include glucose, sucrose, fructose, starch, starch hydrolyzate, molasses and other hydrocarbons which may be used, for example, in an amount 0.5—5.0%. As the nitrogen sources, it is preferred to use, for example, ammonium sulfate, ammonium phosphate, ammonium carbonate, ammonium acetate, and various other inorganic and organic compounds containing ammonia; peptone, yeast extract, corn steep liquor, casein hydrolyzate and various other organic substances containing nitrogen in an amount of 0.5—2.0%. Various inorganic substances such as potassium dihydrogen phosphate, dipotassium hydrogen phosphate, magnesium sulfate, manganese sulfate and the like may be used as inorganic substances in a preferred amount of 0.005 to 0.5%. The culturing may preferably be effected under aerobic conditions with shaking or with shaking and aeration. The culturing temperature is preferably 25—37°C and the culturing time may preferably be 16—40 hours. In this manner, it is possible to accumulate a large amount of glutathione in the microbial cells.

After completion of the culturing, the glutathione accumulated in the microbial cells may be extracted, for example, with water by heating to 100°C. The isolation of glutathione from the extracted solution may be effected by suitable methods, for example, by treating the extracted solution with a suitable ion exchange resin which is known *per se*.

According to another feature of the present invention, there is provided a process for the preparation of glutathione which comprises culturing a microoragnism as hereinbefore defined in a medium therefor to accumulate a glutathione synthesizing enzyme system, if desired separating the enzyme system from the culture medium, reacting L-glutamic acid, L-cysteine and glycine in the presence of said enzyme system whereby to produce glutathione and separating the glutathione obtained from the reaction mixture. The glutathione synthesizing enzyme system may for example be in the form of a "material obtained by treating the microbial cells".

In this case, the expression "a material obtained by treating the microbial cells" denotes, for example, dried microbial cells, a cell-free extract obtained by ultrasonic treatment of the microbial cells, an enzyme obtained by purifying such a cell-free extracted solution, as well as immobilized microbial cells or immobilized purified enzyme obtained by immobilizing the microbial cells or purified enzyme in a conventional manner (e.g. by entrapping with polyacrylamide gel or carrageenan gel).

This process is based upon the discovery that the microbial cells obtained by culturing a microorgansim of the present invention may be used as an enzyme source for the formation of glutathione from L-glutamic acid, L-cysteine and glycine.

The concentrations of L-glutamic acid, L-cysteine and glycine in the substrate solution are preferably 5—50 mM, 5—50 mM and 50—100 mM respectively. The reaction may be effected at a pH of 6—9, preferably 7—8.5 and conveniently at a temperature of 20—50°C, preferably, 30—37°C.

In order to promote the enzymatic reaction, it is preferred to carry out the reaction in the presence of an adenosine-5'-triphosphate (ATP) regeneratoin system. In this connection, it is possible to use the reactions effected by various enzymes present in the microorganism used for the process of the present invention, for example, acetate kinase, enzymes for glycolysis, carbamylphosphate kinase, pyruvate kinase and the like as the ATP regeneration system. For example, the reaction with acetate kinase may with advantage be used for this purpose in the presence of for example magnesium ions e.g. 5—20 mM (in the form of magnesium salts e.g. magnesium sulfate, magnesium chloride, etc.), ATP e.g. 2—5 mM and acetylphosphate e.g. 5—10 mM in the substrate solution.

After completion of the reaction, the glutathione accumulated in the reaction mixture may be isolated and purified in conventional manner, for example, using an appropriate ion exchange resin. For example, the pH of the reaction solution may be adjusted to 3 with sulfuric acid and the solution passed through a cation exchange resin such as Diaion PK-228 H$^+$ (commercially available from Mitsubishi Kasei Kogyo K.K., Tokyo) to adsorb glutathione onto the resin, from which glutathione is eluted with 0.5 M ammonium hydroxide. The pH of eluate is adjusted to 4.5 with sulfuric acid and passed through an anion exchange resin such as Duolite A2 CH$_3$COO— form (commercially available from Diamond Alkali Co., U.S.A.) to adsorb glutathione onto the resin. The adsorbed glutathione is eluted with 0.5 M sulfuric acid, 50% ethanol is added to the eluate to give crystals of glutathione, which may then be isolated for example by filtration.

The microorganisms of the present invention may be employed as starting material in the preparation of further microorganisms as described in our EP—A—71,486, or may be used as described herein in the preparation of glutathione.

The following non-limiting Examples illustrate the present invention:—

Example 1

Preparation of a revertant strain of *E. coli* RC912:

*E. coli* B 355 (ATCC 23226), which is active upon -glutamyl-L-cysteine synthetase (E.C. 6.3.2.2., hereinafter referred to as GSH-I) and glutathione synthetase (E.C. 6.3.2.3., hereinafter referred to as GSH-II), is treated to induce a cysteine-requiring strain and a methylglyoxal-resistant strain. The induction of the mutant strains was effected in conventional manner by using N-methyl-N'-nitro-N-nitrosoguanidine (NTG). A portion of the treated strain was cultured using a minimum medium containing L-cysteine ($2 \times 10^{-5}$ M) and having a composition of $K_2HPO_4$ (0.7%), $KH_2PO_4$ (0.3%), $(NH_4)_2SO_4$ (0.1%), $MgSO_4.7H_2O$ (0.01%) and glucose (0.5%) [pH about 7.0; hereinafter referred to as DM medium] at a temperature of 37°C for 24 hours with shaking to obtain smaller colonies, from which a crysteine-requiring strain was obtained and cultured pure under the same conditions. Separately, the remaining portion of the strain, after induction treatment, was cultured under similar conditions by using a DM medium containing methylglyoxal ($2 \times 10^{-3}$ M) to obtain larger colonies, from which a methylglyoxal-resistant strain was obtained. The methylglyoxal-resistant strain thus obtained was then transferred to the above-mentioned DM medium containing the cysteine-requiring strain ($10^{-7}$ cells/ml) for culturing under the same conditions to obtain colonies having no halo around them, from which the mutant strain *E. coli* C 912 was obtained. This strain which is deficient in the productivity of GSH-I was then treated with NTG in a similar manner to that described above to induce mutation, followed by culturing under the same conditions as described above by using a DM medium containing 8-hydroxyquinoline ($2 \times 10^{-4}$ M) to obtain colonies, from which a mutant strain was obtained which synthesises both GSH-I and GSH-II but which GSH-I is insensitive to feedback inhibition by glutathione, *E. coli* RC 912 (FERM-BP No. 47).

Example 2

The strains shown in the following Table 1 were cultured at 37°C for 16 hours with shaking using 2 ml of a medium having the composition: glucose (0.5%), potassium dihydrogen phosphate (0.3%), dipotassium hydrogen phosphate (0.7%), magnesium sulfate .7H$_2$O (0.01%) and ammonium sulfate (0.1%) [pH 7.0]. After completion of the culturing, the cultured broths were centrifuged (8000 r.p.m./10 min.) to collect the microbial cells which were washed with a 0.85% physiological solution of sodium chloride and extracted with hot water (1 ml; 100°C). The amount of glutathione in the extracted solution was measured to determine the glutathione accumulated in umoles per 1 gram of the microbial wet cells. The results are shown in Table 1.

### TABLE 1

| Strain | Accumulated glutathione (μmole/1 g of wet cells) |
|---|---|
| *Escherichia coli* RC 912 of the present invention (FERM-BP No. 47) | 2.8 |
| *Escherichia coli* B 355 (wild strain) | 1.8 |

Example 3

The culturing and the collection of the microbial cells were effected in a similar manner to that described in Example 2 and the ultrasonic treatment (90 KHz/5 min.) of the microbial cells was carried out to obtain a cell-free extracted solution, of which 0.05 ml was then added to a 50 mM tris-buffer solution (pH 7.5; 9.95 ml) containing L-glutamic acid (25 mM), L-cysteine (25 mM), glycine (50 mM), magnesium chloride (10 mM), acetylphosphate (10 mM) and ATP (5 mM) to effect the enzymatic reaction at 37°C for one hour. After completion of the reaction, the amount of glutathione formed in the reaction solution was measured to determine the accumulated glutathione per one mg of protein. The results are shown in the following Table 2.

### TABLE 2

| Strain | Accumulated glutathione (μmole/1 mg of protein) |
|---|---|
| *Escherichia coli* RC 912 of the present invention (FERM-BP No. 47) | 0.54 |
| *Escherichia coli* B 355 (wild strain) | 0.11 |

**Claims**

1. A mutant strain of *Escherichia coli*, which synthesises a glutathione synthesizing enzyme system comprising γ-glutamyl-L-cysteine synthetase and glutathione synthetase, and said γ-glutamyl-L-cysteine synthetase having a reduced or absent sensitivity to inhibition by glutathione.

2. A mutant strain as claimed in claim 1, designated *Escherichia coli* RC912 (FERM-BP No. 47).

3. A process for the preparation of a strain as defined in claim 1 which comprises subjecting a strain of *Escherichia coli* deficient in the production of γ-glutamyl-L-cysteine synthetase to mutagenesis and isolating the desired strain by selection in the presence of 8-hydroxyquinoline.

4. A process as claimed in claim 3 wherein said strain deficient in the production of γ-glutamyl-L-cysteine synthetase has been prepared by the following steps:

(i) inducing a cysteine-requiring strain and a methylglyoxal-resistant strain in a starting strain of *Escherichia coli* by mutation techniques known *per se*;

(ii) co-culturing the cysteine-requiring strain and the methylglyoxal-resistant strain so obtained in the presence of methylglyoxal; and

(iii) isolating methylglyoxal resistant colonies which are not surrounded by a halo of cysteine-requiring cells.

5. A process for the preparation of glutathione, which comprises cluturing strain as defined in claim 1 or 2 in a medium to accumulate glutathione in the cultured broth and recovering the accumulated glutathione therefrom.

6. A process as claimed in claim 5, in which the recovery is effected by extracting glutathione with water from the microbial cells.

7. A process as claimed in claim 5 or 6, in which the culturing is effected at a pH of from 6 to 9 and a temperature of from 25 to 37°C for 16—40 hours under aerobic conditions.

8. A process for the preparation of glutathione, which comprises culturing a strain as defined in claim 1 or 2 in a medium to accumulate a γ-glutamyl-L-cysteine synthesizing enzyme system in the cultured broth, separating the enzyme system from the cultured broth, placing the same into contact with a substrate solution comprising L-glutamic acid, L-cysteine and glycine to carry out an enzymatic reaction for formation of glutathione, and separating glutathione from the reaction solution.

9. A process as claimed in claim 8, in which the enzyme system comprises γ-glutamyl-L-cysteine synthetase and glutathione synthetase.

10. A process as claimed in claim 6, in which the enzyme system is contained in microbial cells separated from the cultured broth or a cell-free extract of the cultured broth.

11. A process as claimed in claim 8, in which the enzyme system comprises purified enzymes.

12. A process as claimed in any of claims 8—11 in which the reaction is effected by using acetate kinase contained in the microbial cells in the presence of magnesium ion, ATP and acetylphosphate.


**Patentansprüche**

1. Mutantenstamm von *Escherichia coli*, der ein Glutathion synthetisierendes Enzymsystem synthetisiert, welches die γ-Glutamyl-L-cystein Synthetase und die Glutathion Synthetase umfaßt, wobei die γ-Glutamyl-L-cystein Synthetase eine verminderte oder fehlende Sensitivität gegenüber einer Inhibition durch Glutathion aufweist.

2. Mutantenstamm nach Anspruch 1, mit der Bezeichnung *Escherichia coli* RC912 (FERM-BP No. 47).

3. Verfahren zur Herstellung eines Stammes gemäß Anspruch 1, umfassend die Mutagenese eines *Escherichia coli*-Stammes mit fehlender γ-Glutamyl-L-cystein Synthetaseproduktion und die Isolierung des gewünschten Stammes durch Selektion in Gegenwart von 8-Hydroxychinolin.

4. Verfahren nach Anspruch 3, worin der Stamm mit fehlender γ-Glutamyl-L-cystein Synthetaseproduktion durch folgende Schritte hergestellt wurde:

(i) Induzierung eines Cystein-abhängigen Stammes und eines Methylglyoxylat-resistenten Stammes in einen Ausgangsstamm von *Escherichia coli* mil Hilfe von bekannten Mutationstechniken;

(ii) gemeinsame Kultivierung des so erhaltenen Cysteinabhängigen Stammes und des Methylglyoxylat-resistenten Stammes in Gegenwart von Methylglyoxylat; und

(iii) Isolierung der Methylglyoxylat-resistenten Kolonien, welche nicht von einem Halo aus Cystein-abhängigen Zellen umgeben sind.

5. Verfahren zur Herstellung von Glutathion, welches die Kultivierung eines Stammes gemäß Anspruch 1 oder 2 in einem Medium umfaßt, wobei Glutathion im Kulturmedium angereichert wird und man das angereicherte Glutathion daraus isoliert.

6. Verfahren nach Anspruch 5, wobei die Isolierung durch Extraktion von Glutathion mit Wasser aus den Zellen der Mikroorganismen erfolgt.

7. Verfahren nach Anspruch 5 oder 6, worin die Kultivierung bei einem pH-Wert von 6 bis 9 und bei einer Temperatur von 25—37°C in 16—40 Stunden unter aeroben Bedingungen erfolgt.

8. Verfahren zur Herstellung von Glutathion, umfassend die Kultivierung eines Stammes gemäß Anspruch 1 oder 2 in einem Medium, zur Anreicherung eines γ-Glutamyl-L-cystein synthetisierenden Enzymsystems in einem Kulturmedium, Isolierung des Enzymsystems aus dem Kulturmedium, in Kontakt bringen desselben mit einer Substratlösung, welche L-Glutaminsäure, L-Cystein und Glycin umfaßt, wobei eine enzymatische Reaktion zur Bildung von Glutathion erfolgt, und Abtrennung des Glutathions aus der Reaktionslösung.

9. Verfahren nach Anspruch 8, wobei das Enzymsystem γ-Glutamyl-L-cysteinsynthetase und Glutathion Synthetase umfaßt.

10. Vefahren nach Anspruch 6, wobei das Enzymsystem in den Zellen der Mikroorganismen getrennt vom Kulturmedium oder in einem zellfreien Extrakt des Kulturmediums vorliegt.

11. Verfahren nach Anspruch 8, wobei das Enzymsystem gereinigte Enzyme umfaßt.

12. Verfahren nach einem der Ansprüche 8—11, wobei die Reaktion mit Hilfe von Acetatkinase die in den Zellen der Mikroorganismen vorliegt, in Gegenwart von Magnesiumionen, ATP und Acetylphosphat durchgeführt wird.

**Revendications**

1. Souche mutante de *Escherichia coli*, qui synthétise un système enzymatique de synthèse du glutathion, comprenant de la γ-glutamyl-L-cystéine-synthétase et de la glutathion-synthétase, ladite γ-glutamyl-L-cystéine-synthétase présentant une sensibilité réduite ou nulle à l'inhibition par le glutathion.

2. Souche mutante suivant la revendication 1, désignée sous le nom de *Escherichia coli* RC 912 (FERM-BP No. 47).

3. Procédé de préparation d'une souche suivant la revendication 1, qui consiste à soumettre une souche de *Escherichia coli* déficiente en production de γ-glutamyl-L-cystéine-synthétase à une mutagénèse et à isoler la souche désirée par sélection en présence de 8-hydroxyquinoléine.

4. Procédé suivant la revendication 3, dans lequel la souche déficiente en production de γ-glutamyl-L-cystéine-synthétase a été préparée par mise en oeuvre des étapes suivantes:

(i) induction d'une souche dépendante en cystéine et d'une souche résistante au méthylglyoxal dans une souche de départ de *Escherichia coli* par des techniques de mutation en elles-mêmes connues;

(ii) culture conjointe de la souche dépendante en cystéine et de la souche résistante au méthylglyoxal ainsi obtenue en présence de méthylglyoxal; et

(iii) séparation de colonies résistantes au méthylglyoxal qui ne sont pas entourées par une auréole de cellules dépendantes en cystéine.

5. Procédé de préparation de glutathion, qui consiste à activer une souche suivant la revendication 1 ou 2 dans un milieu pour accumuler du glutathion dans le bouillon de culture, et à séparer de ce bouillon le glutathion accumulé.

6. Procédé suivant la revendication 5, dans lequel la séparation est effectuée par extraction du glutathion des cellules microbiennes avec de l'eau.

7. Procédé suivant la revendication 5 ou 6, dans lequel la culture est effectuée à un pH de 6 à 9 et à une température de 25 à 37°C pendant 16 à 40 heures dans des conditions aérobies.

8. Procédé de préparation de glutathion, qui consiste à cultiver une souche suivant la revendication 1 ou 2 dans un milieu pour accumuler un système enzymatique de synthèse de γ-glutamyl-L-cystéine dans le bouillon de culture, à séparer le système enzymatique du bouillon de culture, à mettre ce système enzymatique en contact avec une solution de substrat comprenant de l'acide L-glutamique, de la L-cystéine et de la glycine pour provoquer une réaction enzymatique destinée à la formation du glutathion, et à séparer le glutathion de la solution réactionnelle.

9. Procédé suivant la revendication 8, dans lequel le système enzymatique comprend de la γ-glutamyl-L-cystéine-synthétase et de la glutathion-synthétase.

10. Procédé suivant la revendication 6, dans lequel le système enzymatique est présent dans des cellules microbiennes séparées du bouillon de culture ou bien dans un extrait acellulaire du bouillon de culture.

11. Procédé suivant la revendication 8, dans lequel le système enzymatique consiste en enzymes purifiés.

12. Procédé suivant l'une quelconque des revendications 8 à 11, dans lequel la réaction est conduite en utilisant de l'acétate-kinase présente dans les cellules microbiennes, et présence d'ions magnésium, d'ATP et de phosphate d'acétyle.